# EUROPEAN PATENT APPLICATION

(11) **EP 1 967 129 A1**
(43) Date of publication of application: **10.09.2008**
(21) Application number: 08004383.9
(22) Date of filing: 10.03.2008
(51) Int. Cl.: A61B 1/00, A61B 5/00, G01N 29/24, G01N 21/47

(54) **Medical apparatus obtaining information indicative of internal state of an object based on interaction between sound waves and light**

(30) Priority: 08.03.2007 JP 2007059103
(71) Applicant: Olympus Medical Systems Corp., Tokyo 151-0072 (JP)
(72) Inventor: Igarashi, Makoto, Tokyo 151-0072 (JP); Kazuhiro, Gono, Tokyo 151-0072 (JP)
(74) Representative: von Hellfeld, Axel

(57) **Abstract**

A medical apparatus acquires information indicating an internal state of a region being examined of an object based on a modulated component of light caused by interaction with ultrasound waves in the region. In the apparatus, light radiating component (4,52f,52a,53a,21b,22e1) has a light radiating member (22g1) radiating the light toward the region of the object, and light detecting components (22g2,22e2,21c,53b) have a light detecting member (22g2) detecting light that has been transmitted through the region of the object. The light radiating and detecting components are supported by support components (22h,59,22f1,22f2) configured to be opposed to each other with the region located between the light radiating and detecting members. An ultrasound emitting component (22d) emits the ultrasound waves toward the region from a direction different from a radiating direction of the light radiated from the light radiating member. The emitted ultrasound waves modulate the radiated light.

## Description

### Background of the Invention

### (The field of the Invention)

The present invention relates to a medical apparatus and in particular, to a medical apparatus that detects a modulated component of light which is due to interaction between sound waves and light In the inside of an object to be observed and obtains information indicative of the internal medical state of the object based on the modulated component of the light.

### (Related art)

Various types of medical modalities are known. An optical imaging apparatus, which has been highlighted recently, and the conventionally used endoscope are among them.

Of these instruments, the endoscope has been widely used In the medical field as well as the industrial field. Especially, in the medical field, the endoscope is used to observe the tissue in patient's body cavities and others and perform various treatments.

Meanwhile, medical modalities which obtain tomographic images of objects based on an optical Imaging technique are also known recently. The optical imaging technique Includes various types of techniques such as optical CT (computed tomography), optical coherence tomography, and ultrasound modulated optical tomography. In the medical field, these kinds of medical imaging have attracted attention as a technique to observe various symptoms of objects in terms of a simple and noninvasive manner. Additionally, a system in which an optical imaging apparatus and an endoscope are implemented in a combined manner is also known.

The ultrasound modulated optical tomography is imaging that uses interaction between the ultrasound waves and the light. In an-apparatus for performing this ultrasound modulated optical tomography, ultrasound and light are radiated into a biological body of an object so as to cause the light to pass an in-body region in which the radiated ultrasound is locally converged. The light is subjected to modulation or scattering when passing the region to generate an optical component in which the modulation or scattering is reflected. The optical component is acquired as characteristic information, on which data of tomographic images of the body inside can be obtained. By way of example, Japanese Patent Laid-open Publication No. 2000-88743 discloses an optical measurement apparatus which provides tomographic images of a patient's body using the ultrasound modulated optical tomography.

However, the foregoing optical measurement apparatus is silent about means for radiating the ultrasound and light so that the ultrasound is converged in a body's inside region while the light is directed along a direction passing the region.

### Summary of the Invention

The present invention has been made in consideration of the foregoing conventional situation, and an object of the present invention is to provide a medical apparatus which has a positional and directional setting mechanism which allows the ultrasound modulated optical tomography to be performed accurately at a desired deep position inside the body.

In the present invention, a medical apparatus is provided for acquiring Information Indicative of an internal state of a region being examined of an object based on a modulated component of light caused by interaction with ultrasound waves in the region. The apparatus comprises light radiating means (4, 52f, 52a, 53a, 21b, 22e1) having a light radiating member (22g1) for radiating the light toward the region of the object; light detecting means (22g2, 22e2, 21c, 53b) having a light detecting member (22g2) for detecting light that has been transmitted through the region of the object, the transmitted light being the light radiated from the light radiating member; support means (22h, 59, 22f1, 22f2) for supporting both the light radiating member and the light detecting member so as to be opposed to each other with the region located between the light radiating and detecting members; and ultrasound emitting means (22d, 7) having an ultrasound emitting member (22d) for emitting the ultrasound waves toward the region of object from a direction different from a radiating direction of the light radiated from the light radiating member, the emitted ultrasound waves modulating the radiated light.

The support means supports the light radiating and detecting members in the state where a region to be examined of an object is securely located between both members. Hence, the ultrasound modulated optical tomography can be performed accurately at desired deep positions inside the object body.

### Brief Description of the Drawings

In the accompanying drawings:
Fig. 1 is a block diagram outlining essential components of a biological observation system in which an endoscope serving as a medial apparatus according to a first embodiment of the present invention is adopted;
Fig. 2 is a sectional view of the distal end of the endoscope, which is taken along an X-axis direction in Fig. 1;
Fig. 3 is a perspective view showing a practical configuration of the distal end of the endoscope;
Fig. 4 is a block diagram outlining essential components of a biological observation system in which an endoscope serving as a medial apparatus according to a first modification of the first embodiment is adopted;
Fig. 5 is a block diagram outlining essential components of a biological observation system in which an endoscope serving as a medial apparatus according to a second modification of the first embodiment is adopted;
Fig. 6 is a block diagram outlining essential components of a biological observation system in which an endoscope serving as a medial apparatus according to a third modification of the first embodiment is adopted;
Fig. 7 is a block diagram outlining essential components of a biological observation system in which an endoscope serving as a medial apparatus according to a fourth modification of the first embodiment is adopted;
Fig. 8 is a partial perspective view outlining essential components of a biological observation system in which an endoscope serving as a medial apparatus according to a second embodiment of the present invention is adopted, the endoscope having a plurality of pairs of needles;
Fig. 9 is a partial perspective view outlining essential components of a biological observation system in which an endoscope serving as a medial apparatus according to a third embodiment of the present invention is adopted, the endoscope is loaded with a sheath;
Fig. 10 illustrates various ultrasound converged positions within the inner space of a cap of the sheath;
Fig. 11 is a side view showing a therapeutic instrument serving as a medical apparatus according to a fourth embodiment of the present invention;
Fig. 12 is a partial view explaining a practical structure of the distal portion of the therapeutic instrument; and
Fig. 13 explains the motions of the distal portion of the therapeutic instrument when being manually operated.

### Detailed Description of the Preferred Embodiments

Referring to the accompanying drawings, various embodiments of the present invention will now be described.

### (First embodiment)

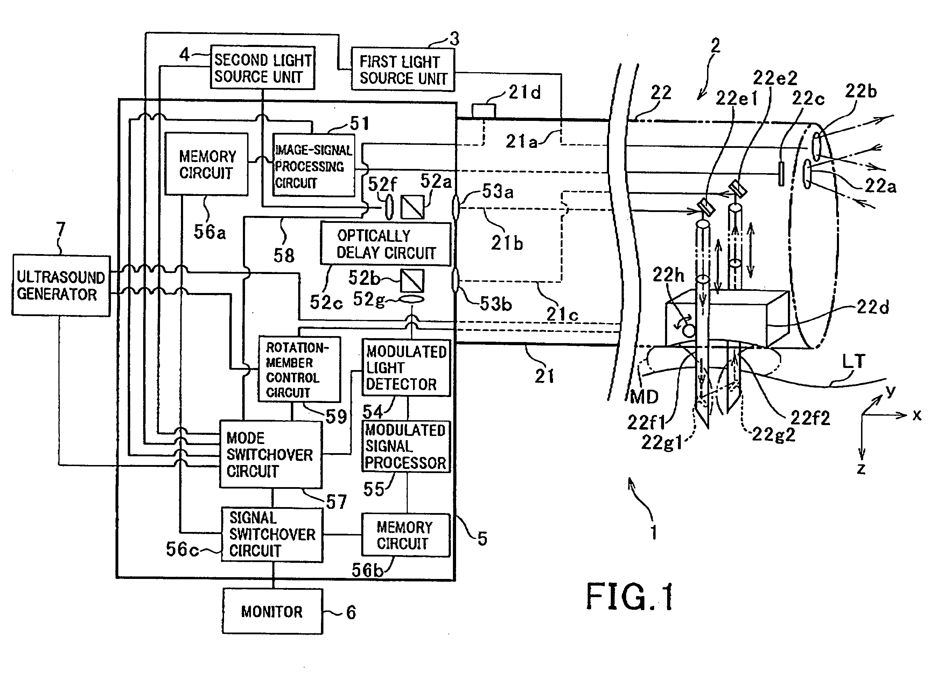

Referring to Figs. 1-3, a biological observation system 1, to which the medical apparatus according to the first embodiment of the present invention, will now be described.

Fig. 1 shows an outlined configuration of the biological observation system 1. This biological observation system 1 is provided with, as its essential components, an endoscope 2, a first light source unit 3, a second light source unit 4, a controller 5, a monitor 6, and an ultrasound generator 7.

Of these, the endoscope 2 can be inserted into body cavities of an object (i.e., patient) to be observed and is formed to observe and treat a region to be examined of body tissue LT existing in a body cavity. The first light source unit 3 is configured to emit light for illuminating the region to be examined in the conventional observation mode. The second light source unit 4 is configured to emit light (light beam) for observing the region to be examined in an ultrasound modulated optical tomography mode which will be stated later. The controller 5 is configured to process electrical signals and returned light both outputted from the endoscope 2 and produce image signals based on the electrical signal and light from endoscope 2. The produced image signals are sent, as signals for images, to the monitor 6 to present the produced image signal. The ultrasound generator 7 is operative under the control of the controller 5 and is configured to output ultrasound waves toward the body tissue via the endoscope 2.

As shown in Fig. 1, the endoscope 2 comprises an elongated and flexible insertion tube 21, which is connectable to the controller 5 and can be inserted into a patient's body cavity and a distal member 22 secured to a distal end of the insertion tube 21. For using the present biological observation system 1, a space between the distal member 22 of the endoscope 2 and a patient's body tissue LT is filled with a medium MD for transmitting the ultrasound therethrough, which is for example water contained in a bag. In the figure shown in Fig. 1, for the sake of a simplified explanation, the orthogonal coordinate system is given in which the X-axis is assigned to the longitudinal direction of the insertion tube 21. When it is needed to explain the directions, the X-, Y- and Z-axes are used.

Electrical cables and light guide cables are inserted through the insertion tube 21. These cables include, in addition to various types of electrical signal lines for transmitting electrical signals, light guide cables 21a, 21b and 21c. Of these, the first light guide cable 21a is for transmitting, to the distal member 22, light emitted from the first light source unit 3. The second light guide cable 21b is for transmitting, to the distal member 22, light emitted from the second light source unit 4 to the distal member 22. The third light guide cable 21c is for transmitting, to the controller 5, reflected light outputted from the distal member 22.

A mode switch 21d, which is used by a user to make switchovers between the conventional observation mode and the ultrasound modulated optical tomography mode, is arranged at an X-axis directional base-side position on the outer surface of the insertion tube 21. The base side means "leftward in Fig. 1." It is not always limited to the configuration where the mode switch 21d is arranged on the endoscope 2, but this switch 21d may be arranged on, for example, a not-shown operation panel prepared for the controller 5.

The distal member 22 is equipped with an objective optical system 22a, an illuminating optical system 22b, and an imaging element 22c. Both optical systems 22a and 22b are placed to have an optical axis which is parallel with the insertion axis direction (longitudinal direction) of the rigid distal end of the endoscope 2. The imaging element 22c is located at an image formation position of the objective optical system 22a.

The illuminating optical system 22b is to function in the conventional observation mode. This system 22b receives the illuminating light emitted from the first light source unit 3 and then transmitted through the light guide cable 21a, and outputs the illuminating light toward the body tissue LT. Hence the body tissue LT is illuminated by the illuminating light.

The imaging element 22c is provided with a CCD (charge-coupled device) and made active in the conventional observation mode. This imaging element 22c produces imaging signals depending on images of the body tissue LT image-formed by the objective optical system 22a and outputs the imaging signals to the controller 5.

The distal member 22 is further equipped with an ultrasound emitting unit 22d which emits ultrasound waves under the control of the ultrasound generator 7 and various optical mirrors and drive mechanisms. These optical mirrors and drive mechanisms include a first total reflection mirror 22e1, a second total reflection mirror 22e2, optically transparent needles 22f1 and 22f2, and a rotation member 22h. Of these components, the total reflection mirror 22e1 is located adjacently to the light-output-side end of the light guide cable 21b. Meanwhile the second total reflection mirror 22e2 is located adjacently to the light-input-side end of the light guide cable 21c. The rotation member 22h is under the control of the controller 5 and is driven to move the respective needles 22f1 and 22f2 in the Z-axis direction.

The ultrasound emitting unit 22d is configured to perform electronic scanning of ultrasound waves to be emitted therefrom. Though not shown in Fig. 1, this ultrasound emitting unit 22d is provided with a backing material, an ultrasound transducer that emits ultrasound waves in response to control of the ultrasound generator 7, matching layers, and an acoustic lens converging the ultrasound waves in, for example, the X-axis direction perpendicular to the inserting direction of the endoscope 2. These components are stacked on one another in a casing. The ultrasound emitting unit 22d responds to time delay control performed in the ultrasound generator 7 to change the radiating direction and the convergent position of the ultrasound waves emitted from the ultrasound transducer, so that the ultrasound waves can be spatially scanned along a desired region to be examined in an electrical manner.

The total reflection mirror 22e1 receives the radiating light sent through the light guide cable 21b to reflect the received light in the Z-axis direction.

Of the needles 22f1 and 22f2, the needle 22f1 is a hypodermic needle having a cylindrical inner space formed therethrough and a light radiating member placed in the inner space. This needle 22f1 has an end portion which can be inserted into the body tissue LT, so that the size and shape of the end portion are set to enable the insertion. In the inner space of the needle 22f1, there is provided a total reflection mirror 22g1, which reflects again, along the Y-axis direction, the radiation light propagated in the inner space of the needle 22f1 from the total reflection mirror 22e1. This total reflection mirror 22g1 in the needle 22f1 is located in place to enable the Y-axial light reflection.

The remaining needle 22f2 is also a hypodermic needle having a cylindrical inner space formed therethrough and a light detecting member placed in the inner space. This needle 22f2 has an end portion which can be inserted into the body tissue LT, so that the size and shape of the end portion are set to enable the insertion. In the inner space of the needle 22f2, there is provided a total reflection mirror 22g2. This mirror 22g2 is located at the same position as that of the foregoing total reflection mirror 22g1 in the same Z-axis direction in the same YZ plane in which the foregoing total reflection mirror 22g1 exists. In other words, both mirrors 22g1 and 22g2 are located to be opposed to each other in the three-dimensional field. Thus the total reflection mirror 22g2 receives the light propagated from the opposed total reflection mirror 22g1 to reflect it upward in the Z-axis direction. This reflected light is propagated through the inner space of the needle 22f2 to enter the total reflection mirror 22e2.

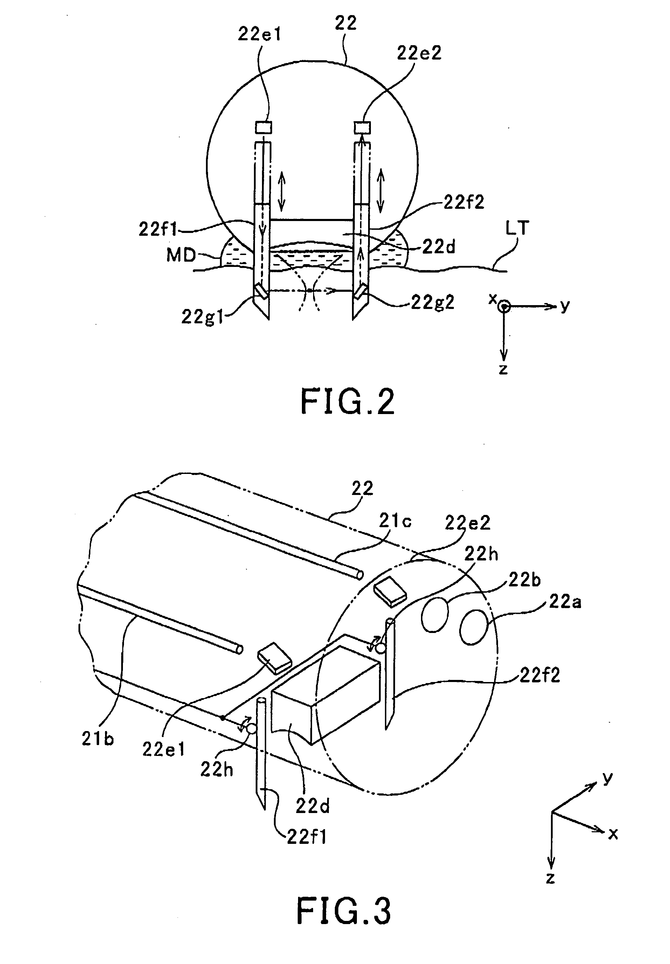

The construction of this distal member 22 is also illustrated in Figs. 2 and 3, in which for the sake of a simplified explanation, the electric wirings and others are partly omitted from being drawn.

Incidentally, in this distal member 22, both needles 22f1 and 22f2 are located such that the axis of the light propagated from the total reflection mirror 22g1 to the total reflection mirror 22g2 is approximately parallel with the Y-axis.

The first light source unit 3 has, for example, a xenon lamp which emits white light. This light source unit 3 is operative in the conventional observation mode and configured to emit the light, serving as illuminating light for illuminating an object to be observed, to a light guide cable 21a under the control of the controller 5.

In contrast, the second light source unit 4 is produced to have, as a light source, a laser light source or an SLD (Super Luminescent Diode), for example. This light source unit 4 is for use in the ultrasound modulated optical tomography mode and responds to control from the controller 5 when emitting light (light beam) for radiation onto a region to be examined, to a light guide cable 58 arranged in the controller.

The controller 5 is provided with, as shown in Fig. 1, an image-signal processing circuit 51, beam splitters 52a and 52b, an optically delay circuit 52c, lenses 52f and 52g, collecting lenses 53a and 53b, a modulated light detector 54, a modulated signal processor 55, memory circuits 56a and 56b, signal switchover circuit 56c, a mode switchover circuit 57, the foregoing light guide cable 58, and a rotation-member control circuit 59.

Of these circuitry components, the image-signal processing circuit 51 is operative under the control of the mode switchover circuit 57, In which the circuit 51 produces a video signal depending on electrical imaging signals from the imaging element 22c and outputs the produced video signal to the memory circuit 56a.

The beam splitter 52a is disposed to receive the radiating light through the lens 52f and split the received light into two light beams in two directions toward the collecting lens 53a and optically delay circuit 52c. The other beam splitter 52b is disposed to receive both the radiating light coming through the optically delay circuit 52c and light coming through the collecting lens 53b so that both two types of incoming light interfere with each other therein. As a result, the interference light is outputted from the splitter 52b to the modulated light detector 54 via the lens 52g.

In this light propagation, the optically delay circuit 52c is placed to optically delay and output the radiating light coming through the beam splitter 52a so that this radiating light agrees with the light coming to the beam splitter 52b through the collecting lens 53b In the light incident timing. Hence the radiating light from the optically delay circuit 52c and the light from the collecting lens 53 are able to enter the beam splitter 52b at the same timing.

The collecting lens 53a, which is inserted in the radiating-light optical system, faces the beam splitter 52a to collect the radiating light from this splitter 52a and provide the collected light to the face of the light-incident-side end of the light guide cable 21b. On the other hand, the collecting lens 53b, which is inserted in the detecting-light optical system, faces the other beam splitter 52b to collect the light which has been propagated through the light guide cable 21c, and provide the collected light to the beam splitter 52b.

The modulated light detector 54 operates under the control of the mode switchover circuit 57, and receives the two-flux interference light coming from the beam splitter 52b. When receiving the interference light, this detector 54 detects modulation characteristic information that shows how much degree the light detected by the needle 22f2 is modulated compared to the radiating light from the second light source unit 4. The modulated light detector 54 outputs the modulation characteristic information to the modulated signal processor 55 as a modulated signal.

The modulated signal processor 55 uses the modulated signal from the detector 54 to produce a video signal depending on the modulated signal and provide the produced video signal to the memory circuit 56b.

Meanwhile, the memory circuit 56a electrically connected to the image-signal processing circuit 51 temporarily memorizes the video signal from this circuit 51 in the conventional observation mode, during which time the circuit 56a outputs the stored video signal to the signal switchover circuit 56c frame by frame in sequence. In contrast, the other memory circuit 56b, which is for the ultrasound modulated optical tomography mode, temporarily memorizes the video signal from the modulated signal processor 55, during which time the circuit 56b outputs the stored video signal to the signal switchover circuit 56c frame by frame in sequence.

The signal switchover circuit 56c is under the control of the mode switchover circuit 57 for both conventional observation mode and ultrasound modulated optical tomography mode. In the former mode, the signal switchover circuit 56c sequentially provides the video signal from the memory circuit 56a to the monitor 6, while in the latter mode, the circuit 56c sequentially provides the video signal from the other memory circuit 56b to the monitor 6.

The mode switchover circuit 57 is configured to respond to operator's manual operations at the mode switch 21d mounted on the endoscope 2. By this operation, this switch 57 is able to selectively specify the observation mode between the conventional observation mode and the ultrasound modulated optical tomography mode.

Practically, manual operations at the mode switch 21d are able to make the observation mode switch over to the conventional observation mode. In this case, the mode switchover circuit 57 orders both the first light source unit 3 and the image signal processing circuit 51 to operate, while ordering the second light source unit 4, modulated light detector 54, rotation-member control circuit 59, and ultrasound generator 7 to stop from working. Hence, in the conventional observation mode, the light from the first light source unit 3 illuminates a body tissue LT to be observed. Thus the imaging element 22c images the illuminated body tissue LT. In this imaging, the mode switchover circuit 57 controls the signal switchover circuit 56c to allow the image signals stored in the memory circuit 56a to be sent to the monitor 6.

In contrast, as need arises, the mode switch 21d is operated to switch the observation mode to the ultrasound modulated optical tomography mode. In this case, the mode switchover circuit 57 operates to start the second light source unit 4, modulated light detector 54, rotation-member control circuit 59, and ultrasound generator 7 and, instead, stop the first light source unit 3 and image-signal processing circuit 51. Thus, in the present ultrasound modulated optical tomography mode, the radiating light is emitted from the radiating needle 22f1 and the ultrasound waves are emitted from the ultrasound emitting unit 22d. Accordingly, the modulation characteristic information is obtained depending on the light received by the other needle 22f2 inserted in the body tissue LT. The mode switchover circuit 57 controls the signal switchover circuit 56c such that the video signal is given to the monitor 6 from the memory circuit 56b.

The rotation-member control circuit 59 is operative under the control of the mode switchover circuit 57 and controls the rotation of the rotation member 22h. This rotation enables the needles 22f1 and 22f2 to positionally be changed in the Z-axis direction, which correspond to changes in protruded amounts of the needles from the distal member 22 in the Z-axis direction. In the present embodiment, using the rotation member 22h, the rotation-member control circuit 59 makes both needles 22f1 and 22f2 move in synchronization with each other in the Z-axis direction. Hence the needles are controlled to protrude by the same amount in the Z-axis direction.

The operations of the biological observation system 1 according to the present embodiment will now be described.

The mode switch 21d is manually operated to set the biological observation system 1 into the conventionally observation mode. In response to this, the mode switchover circuit 57 allows the first light source unit 3 and the image-signal processing circuit 51 to work, but stops the operations of the second light source unit 4, the modulated light detector 54, the rotation-member control circuit 59, and the ultrasound generator 7. Hence the illuminating light emitted from the first light source unit 3 is propagated to the body tissue LT via the light guide cable 21a and the illuminating optical system 22b.

Further, through the objective optical system 22a, the imaging element 22c images the body tissue LT illuminated by the illuminating optical system 22b, and outputs imaging signals of the captured images.

The image-signal processing circuit 51 produces video signals according to the imaging signals from the imaging element 22c and outputs the produced video signals to the memory circuit 56a.

The memory circuit 56a temporarily stores therein the video signals sent out from the image-signal processing circuit 51, during which the circuit 56a outputs in sequence the imaging signals to the signal switchover circuit 56c frame by frame. The signal switchover circuit 56c, which is under the control of the mode switchover circuit 57, outputs to the monitor 6 the video signals sent out from the memory circuit 56a. Accordingly the monitor 6 renders the images of the body tissue LT.

Meanwhile, when the switch 21d is manually operated to the ultrasound modulated optical tomography mode, the mode switchover circuit 57 enables the operations of the second light source unit 4, the modulated light detector 54, the rotation-member control circuit 59, and the ultrasound generator 7. However, the circuit 57 disables the operations of the first light source unit 3 and the image-signal processing circuit 51, instead. This allows the radiating light from the second light source unit 4 to be propagated to the body tissue LT via the beam splitter 52a, lens 53a, light guide cable 21b, total reflection mirror 22e1, and radiating needle 22f1 (with the total reflection mirror 22g1). In parallel to this propagation, the radiating light from the second light source unit 4 is split by the beam splitter 52a to make the split light travel to the optically delay circuit 52c.

The rotation-member control circuit 59 sends a rotation command to the rotation member 22h so as to rotate this member 22h. The rotations of the rotation member 22h, which is engaged with the needles 22f1 and 22f2, causes the needles 22f1 and 22f2 to be moved back and forth in the Z-axis direction. Hence when the needles 22f1 and 22f2 are made to advance forward in the Z-axis direction, the needles 22f1 and 22f2 are forced to protrude from the distal member 22. As a result, both needles 22f1 and 22f2 are pushed into the body tissue LT to reach an inner region to be examined therein, so that the inner region to be examined can be located between both needles 22f1 and 22f2.

Further, the ultrasound generator 7 delay-controls the ultrasound emitting unit 22d such that the ultrasound emitting unit 22d emits ultrasound waves and the emitted ultrasound waves are converged on the optical path of the radiating light from the radiating needle 22f1 through the body tissue LT along the Y-axis direction. Hence, while traveling through the body tissue LT, the light radiated from the needle 22f1 is subjected to modulation on the ultrasound waves in the converged region of the ultrasound waves. Due to this modulation, modulated light which is generated which reaches the detecting needle 22f2.

The ultrasound generator 7 changes the delay time given to the ultrasound emitting unit 22d, so that the ultrasound converged region can be shifted sequentially in the Y-axis direction from a region near the radiating needle 22f1 to a region near to the detecting needle 22f2. In each of the shifted converged regions, the modulated light is produced and detected as above.

As described above, the needles 22f1 and 22f2 are pushed into the body tissue LT in which there is an object to be examined, so the needle 22f1 for radiating the light and needle 22f2 for detecting the modulated light are positionally fixed in the body tissue LT. In this fixed state, along the Y-axial imaginary line connecting the total reflection mirrors 22g1 and 22g2, each point defined by each of the ultrasound converged region is shifted (i.e., scanned on the ultrasound waves) to produce and detect the modulated light.

Moreover, the rotation-member control circuit 59 monitors the operations of the ultrasound generator 7, during which every time it is detected that the ultrasound scanning has completed on each Y-axis line, the circuit 59 operates to shift the ultrasound scanning position in the Z-axis direction in the body tissue LT. For this shift, the rotation member 22h is rotated in a predetermined direction to change the protruded amounts of both needles 22f1 and 22f2 from the distal member 22. This is performed in response to a command from the rotation-member control circuit 59. Thus the Z-axial positions of both radiating needles 22f1 and detecting needle 22f2, i.e., the positions in the depth direction of the tissue body LT, are changed. This allows another Y-axial line is subjected to the radiation of the light from the needle 22f1, and the ultrasound converged region is shifted step by step along the optical path of the radiated light traveling along the new line. The modulated light is therefore detected in each of the ultrasound converged regions on this optical path.

The modulated light detected by the detecting needle 22f2 is propagated to the beam splitter 52b via the total reflection mirror 22g2, total reflection mirror 22e2, light guide cable 21c, and collecting lens 53b. At the timing where the modulated light reaches the beam splitter 52b, the radiating light delayed by the optically delayed circuit 52c also reaches the beam splitter 52b.

Hence both the radiating light from the optically delayed circuit 52c and the modulated light from the collecting lens 53b are made to interfere with each other. The interference light is therefore sent out to the modulated light detector 54.

In the modulated light detector 54, the radiating light via the beam splitter 52b is made reference, with which modulation characteristic information showing how much degree the modulated light is subjected to the modulation from the ultrasound waves is detected. That is, the two-flux light interfered in the beam splitter 52b is used to detect, as the modulation characteristic information, the amount (degree) at which the radiating light is frequency-modulated. Thus, this modulation characteristic information is sent as a modulation signal to the modulated signal processor 55. In combination with the acquisition of the frequency-modulated amount, the modulated light detector 54 also acquires, as the modulation characteristic information, an optical absorbance amount in the modulated light.

In the modulated signal processor 55, a video signal is produced depending on the modulation signal from the modulated light detector 54, and sent to the memory circuit 56b.

While being temporarily memorized in the memory circuit 56b, the video signals are sequentially, frame by frame, outputted to the signal switchover circuit 56c.

From the signal switchover circuit 56c, the monitor 6 receives the video signals. Hence the monitor 6 is able to render tomographic images of the inside of the body tissue LT in which the modulation characteristic information detected by the modulated light detector 54 is reflected.

As stated, the body tissue LT is firmly located between the radiating and detecting needles 22f1 and 22f2 and is subjected to the two-dimensional scanning of the ultrasound/light interacting position. Hence, the ultrasound modulated optical tomography can be performed accurately at desired deep positions inside the object body. By changing the inserted positions of both needles 22f1 and 22f2, another section Is scanned in the same manner for the ultrasound modulated optical tomography.

In the present embodiment, the distal member 22 of the endoscope 2 is provided with the two needles 22f1 and 22f2 which radiates the light through an object being examined and detects the modulated light from the object. However, this is just one example and it is not needed to restrict to such a needle structure. For example, only one needle which is able to radiate the light and detect (receive) the modulated light may be arranged in the distal member 22.

Other various modifications of the biological observation system 1 according to the foregoing embodiment can be provided as follows.

### (First modification)

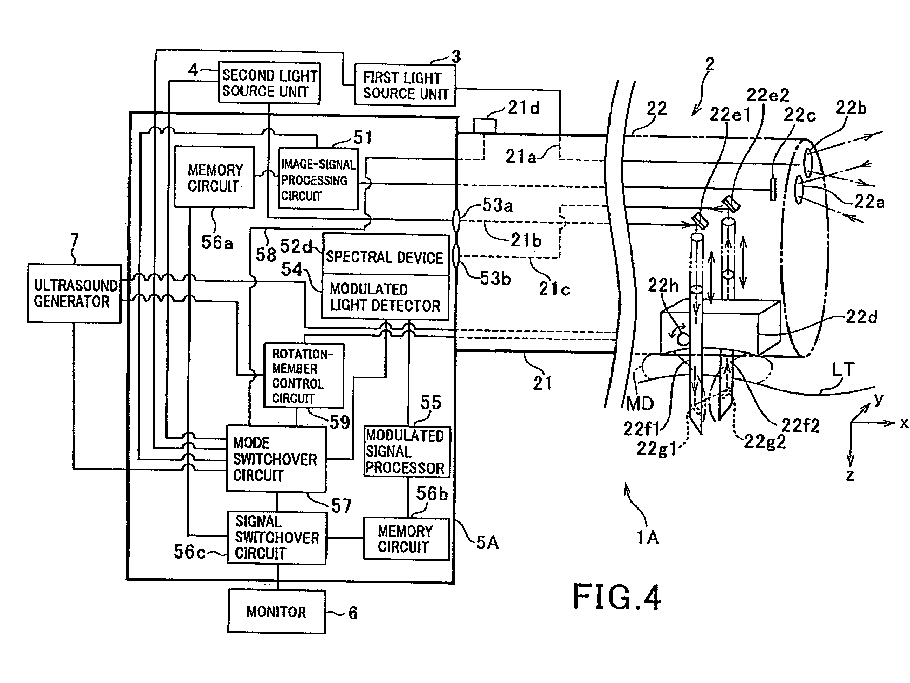

Referring to Fig. 4, a first modification of the foregoing biological observation system 1 will now be described. In the present modification, components which are identical or similar to those in the foregoing embodiment are given the same reference numerals as those used in the first embodiment for the sake of a simplified explanation. This is true of succeeding modifications and embodiments.

The controller 5 arranged in the biological observation system 1 may be modified in terms of acquisition of the modification characteristic information.

Practically, Fig. 4 shows a biological observation system 1A according to the present modification. This system 1A comprises the foregoing endoscope 2, first light source unit 3, second source unit 4, monitor 6, and ultrasound generator 7, and in addition, a controller 5A. The controller 5A is configured as shown in Fig. 4, in which the foregoing beam splitters 52a and 52b and the optically delay circuit 52c, which are shown in Fig. 1, are removed and instead, a spectral device 52d is arranged on the output side of the collecting lens 53b.

Thus, in the controller 5A, the light emitted from the second light source unit 4 is propagated through the light guide cable 58 so as to enter the light guide cable 21b via the collecting lens 53a.

Meanwhile, the modulated light transmitted through the light guide cable 21c enters the controller 5A via the collecting lens 53b, and outputted to the spectral device 52d.

The spectral device 52d comprises any of a diffraction grating, an acoustooptical device, and a liquid crystal tunable filter. Hence the spectral device 52 is able to apply a frequency decomposition process to the modulated light coming via the collecting lens 53b to provide the modulated light detector 54 with the frequency-decomposed modulated light. Thus the detector 54 detects, as the modulation characteristic information, the frequency-decomposed modulated light.

After this detection, the remaining processes are the same as those in the foregoing controller 5. The monitor 6 renders tomographic images of an object being examined in a body tissue LT to be observed, in which the modulation characteristic information detected by the detector 54 is therefore reflected in the tomographic images.

### (Second modification)

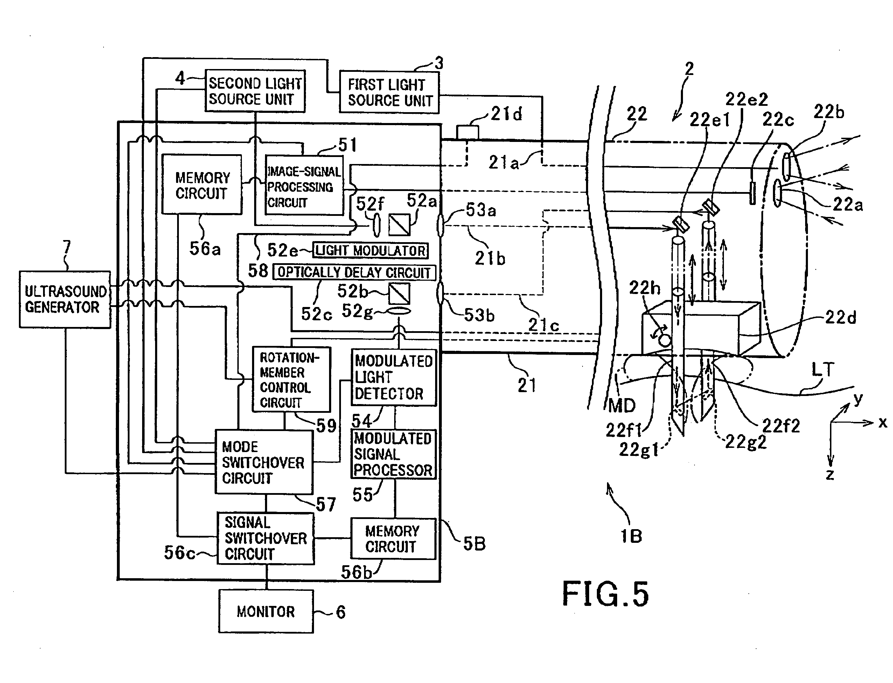

Referring to Fig. 5, a second modification will now be described.

The present modification is also referred to another example of the modulation characteristic information being acquired in the biological observation system. As stated, the type of this modulation characteristic information is not always limited to the frequency-modulated amount, such as Doppler shift frequency Δf. For example, the modulation characteristic information may be an amount showing how much degree the modulated light is subjected to phase modulation. This detection is made by the modification shown in Fig. 5.

Practically, as shown in Fig. 5, the biological observation system 1B comprises a controller 5B in addition to the foregoing endoscope 2, first and second light source units 3 and 4, monitor 6, and ultrasound generator 7. The controller 5B comprises a light modulator 52e inserted between the beam splitter 52a and the optically delay circuit 52c. The remaining components are the same as those in Fig. 1.

The light modulator 52e applies frequency modulation to the light from the beam splitter 52a, and provides the frequency-modulated light to the optically delay circuit 52c.

The frequency-modulated light is provided from the optically delay circuit 52c to the beam splitter 52b with a delay. The beam splitter 52b also receives from the detecting needle 22f2 the modulated light via the light guide cable 21c and the collecting lens 53b. In the beam splitter 52b, the two fluxes of light, which are the frequency-modulated light, serving as reference light, and the detected modulated light, interfere with each other, and the interference light is provided to the modulated light detector 54. This detector 54 uses the two-flux interference light to detect, as the modulation characteristic information, a signal showing how much degree the modulated light is phase-modulated.

The remaining processes in the controller 5B are similar to those in the foregoing controller 5. Thus, the monitor 6 is able to present tomographic images of a body tissue LT by using the signal from the modulated light detector 54.

In the present controller 5B, for the sake of increasing the S/N, another light modulator, which is the same as the foregoing light modulator 52e, may be placed between the beam splitter 52b and the collecting lens 53b.

### (Third modification)

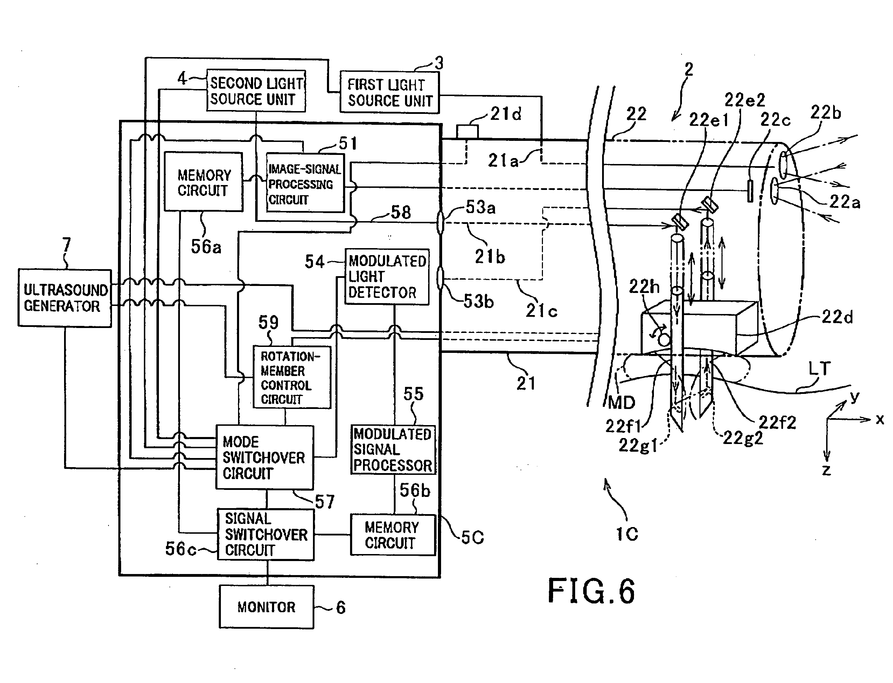

Referring to Fig. 6, a third modification will now be described, which also concerns how to acquire the modulation characteristic information. In this modification, the modulation characteristic information is exemplified as a light-absorbed amount instead of the frequency-modulated amount (such as Doppler shift frequency Δf).

Practically, Fig. 6 shows a biological observation system 1C, which comprises a controller 5C, in addition to the foregoing endoscope 2, first and second light source units 3 and 4, monitor 6, and ultrasound generator 7. The controller 5C has a configuration which is similar to that of Fig. 1 except that the beam splitters 52a and 52b and the optically delay circuit 52c are removed from the configuration.

In the controller 5C, the light emitted from the second light source unit 4 is propagated along the light guide cable 58, and provided to the light guide cable 21b via the collecting lens 53a.

Meanwhile the modulated light, which has been propagated along the light guide cable 21c, is provided to the modulated light detector 54 via the collecting lens 53b.

Thus, the modulated light detector 54 applies a known technique to the received modulated light to detect a light-absorbed amount in the modulated light, which serves as the modulation characteristic information. It is therefore possible that the monitor 6 presents tomographic images of a body tissue LT, which reflect the detected light-absorbed amount.

As a further modification, the modulation characteristic information may be a combination of the frequency-modulated amount and the light-absorbed amount.

### (Fourth modification)

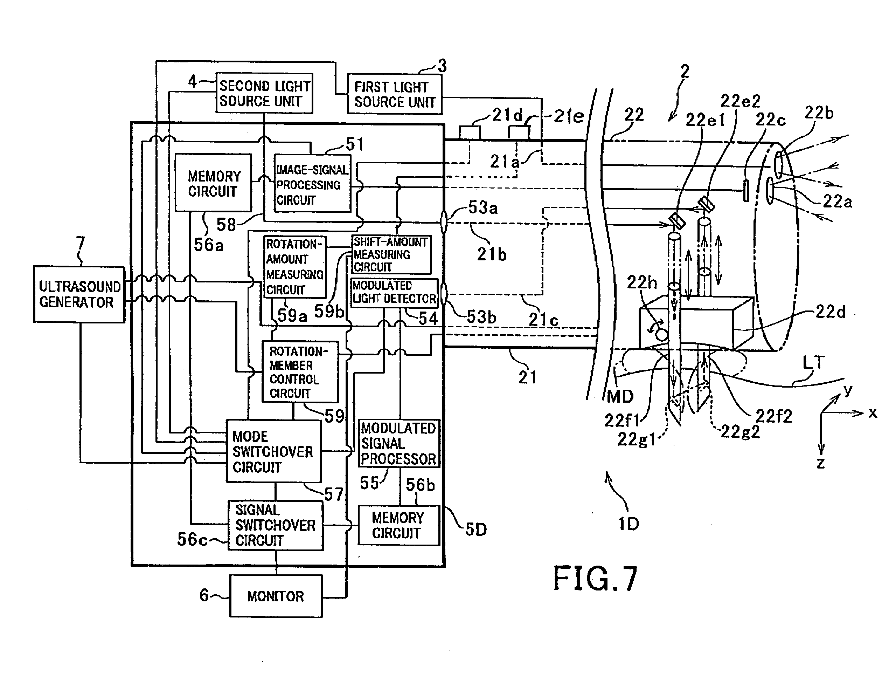

Referring to Fig. 7, a fourth modification will now be described. Fig. 7 shows a biological observation system 1D according to the present modification, in which there is provided a controller 5D that has the capability of detecting an amount of movement of the needles 22f1 and 22f2 to be moved along the Z-axis direction in Fig. 7.

To be specific, as shown in Fig. 7, the biological observation system 1D comprises an endoscope 2D and a controller 5D, in addition to the foregoing first and second light source units 3 and 4, monitor 6, and ultrasound generator 7. The endoscope 2D is structured to have not only the configurations shown in Fig. 6, for example, but also a calibration switch 21e. Meanwhile, the controller 5D comprises the configurations shown by the controller 5C in Fig. 6, a rotation-amount detecting circuit 59a, and a movement-amount detecting circuit 59b.

The calibration switch 21e, which is arranged on the endoscope 2D, is handled (e.g., pressed down) by an operator to give a timing at which the Z-axial positions of the distal ends of both needles 22f1 and 22f2 are detected by using a signal from the rotation-amount detecting circuit 59a, which will be explained later. A signal showing the detected positions is thus given to the movement-amount detecting circuit 59b. For example, when the needles 22f1 and 22f2 touch the surface of a body tissue LT, the calibration switch 21e is pressed down by the operator. Responsively to this operation, it becomes possible to detect, as the needle-moved amount, an insertion amount of the needles inserted into the body tissues LT.

The rotation-amount detecting circuit 59a calculates an amount of rotation of the rotation member 22h by using information indicative of the control commands from the rotation-member control circuit 59 to the rotation member 22h. This detecting circuit 59a then provides the movement-amount detecting circuit 59b with the signal showing the calculated rotation amount.

The movement-amount detecting circuit 59b responds to a command signal from the calibration switch 21e by setting the needle-moved amount to zero. That is, the needle-moved amount becomes zero whenever the command signal is received. The movement-amount detecting circuit 59b uses the signal indicative of the rotated amount, which comes from the rotation-amount detecting circuit 59a, to calculate an amount of needles' movements (for example, insertion) which have performed after the calibration timing. A signal showing this movement amount is sent to from the detecting circuit 59b to the monitor 6.

Hence, the monitor 6 is able to display the moved amounts of the needles 22f1 and 22f2 in an appropriate manner such as numeral values. It is therefore possible to obtain how deeply the needles 22f1 and 22f2 have been inserted into the body tissue LT, whenever the calibration switch 21e is operated in a condition where, for example, the needle's distal ends come in contact with the surface of the body tissue LT. An inserted amount of the needles 22f1 and 22f2 is then displayed by the monitor 6.

### (Second embodiment)

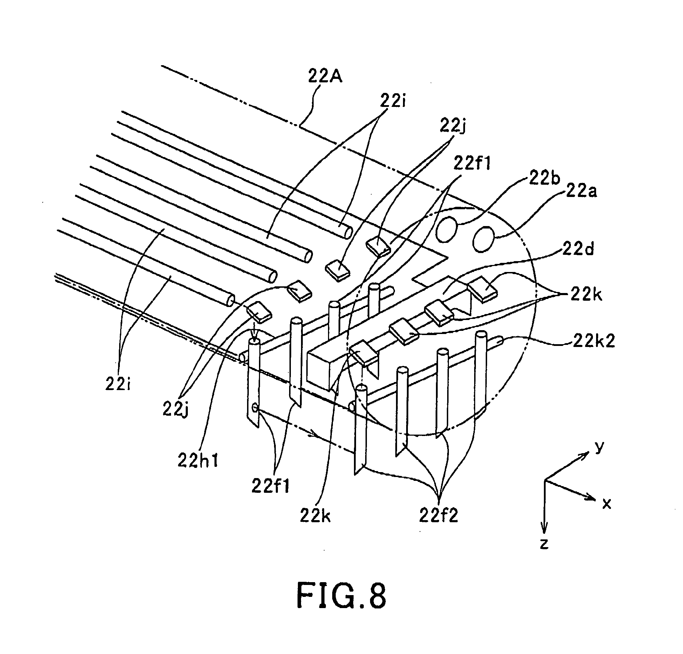

Referring to Fig. 8, a biological observation system according to a second embodiment of the present invention will now be described.

The present biological observation system concerns another configuration for radiating light to a region to be examined and detecting light modulated in the region.

In the biological observation systems according to the first embodiment and its modifications, only one pair of the light-radiating and light-detecting needles 22f1 and 22f2 are provided in the distal member 22 of the endoscope 2. However, this is not a decisive list, but may be developed into a structure shown in Fig. 8, for example. A biological observation system 1E according to the second embodiment Includes a distal member 22A as shown in Fig. 8. The distal member 22A is provided with a plurality of pairs of needles each consisting of paired two needles which are the same as the foregoing paired needles 22f1 and 22f2. For the sake of a simplified explanation, electrical wirings and the like are omitted from being depicted in Fig. 8.

Specifically, as shown in Fig. 8, the distal member 22A comprises four sets of optical systems arranged in the Y-axis direction at intervals. Each optical system includes a light-radiating needle 22f1, a light-detecting needle 22f2, a light guide cable bundle 22i, a half mirror 22j, and a total reflection mirror 22k. The light guide cable bundle 22i has a first light guide cable, which is similar to the foregoing light guide cable 21b, for transmitting the radiating light emitted from the second light source unit 4 to the needle 22f1 and a second light guide cable, which is similar to the foregoing light guide cable 21c, for transmitting the modulated light detected by the needle 22f2 to the controller 5. The half mirror 22j is located in front of the output face of the light guide cable bundle 22i. Moreover, the distal member 22A comprises, in addition to the foregoing ultrasound unit 22d, a rotation member 22h1 on which the four radiating needles 22f1 are placed at intervals and slidable in the Y-axis direction and another rotation member 22h2 on which the four detecting needles 22f2 are placed at intervals and slidable in the Y-axis direction.

The four sets of optical systems have the same optical structure to transmit and receive the light. Hence, in the following description, only one set of optical system will be detailed.

The light propagated along the first light guide cable of the light guide cable bundle 22i in the X-axis direction is reflected by the half mirror 22j to be transmitted in the Z-axis direction.

The radiating needle 22f1 contains a total reflection mirror, though not shown in Fig. 8. Hence this total reflection mirror reflects the light coming from the half mirror 22j to be sent out as a radiating light beam in the X-axis direction.

On the other hand, the detecting needle 22f2 in the distal member 22A also contains therein a not-shown total reflection mirror. This total reflection mirror receives incident modulated light, and reflects the received light toward the total reflection mirror 22k. Incidentally, the not-shown total reflection mirror in the detecting needle 22f2 is located in conformity with the not-shown total reflection mirror in the radiating needle 22f1, so that the modulated light can be detected by the needle 22f2 and transmitted to the total reflection mirror 22k.

The total reflection mirror 22k is located to reflect the modulated light coming from the detecting needle 22f2 toward the input face of the second light guide cable (not shown) in the light guide cable bundle 22i via the half mirror 22j. The modulated light is thus transmitted to the controller 5 through the not-shown second light cable in the cable bundle 22i.

The rotation members 22h1 and 22h2 are eclectically connected to the rotation-member control circuit 59 of the controller 5 by signal lines not shown in Fig. 8. Hence, under the control of the rotation-member control circuit 59, the four needles 22f1 slidable on the rotation member 22h1 are allowed to move in the Z-axis direction synchronously with each other. Likewise, the four needles 22f2 slidable on the rotation member 22h2 are also allowed to move in the Z-axis direction synchronously with each other.

As stated, the endoscope according to the present embodiment is able to three-dimensionally positionally fix the relationship between the ultrasound converged region and the light radiating direction in a region to be examined within a body tissue being observed. Thus, compared to the conventional, the endoscope improves the accuracy of acquisition of the modulation characteristic information in a deep desired region of an object being observed.

The configurations of the second embodiment may also be modified into other various forms.

For example, in the above, the modulated light detected by each needle 22f2 is transmitted though the not-shown second light cable contained in the light guide cable bundle 22i. But this is not a decisive structure. When employing any of the foregoing modifications, the modulated light that has been detected may be transmitted to any of the controllers 5A - 5D to acquire another type of modulation characteristic information.

Further, in the distal member 22A, the radiating and detecting needles 22f1 and 22f2 may be arranged differently from the foregoing such that the needles 22f1 radiate the light in the Y-axis direction and the other needles 22f2 detects the modulated light in the Y-axis direction.

In addition, the total reflection mirror arranged in each paired needles 22f1 and 22f2 may be composed of a micro-mirror, which is driven a driver to be arranged in the controller 5 so as to change the light radiating direction and the modulated-light receiving (detecting) direction in a controlled manner. This enables the light to be variably transmitted and received between the needles, resulting in a wider ultrasound scanning range without moving the endoscope distal end.

### (Third embodiment)

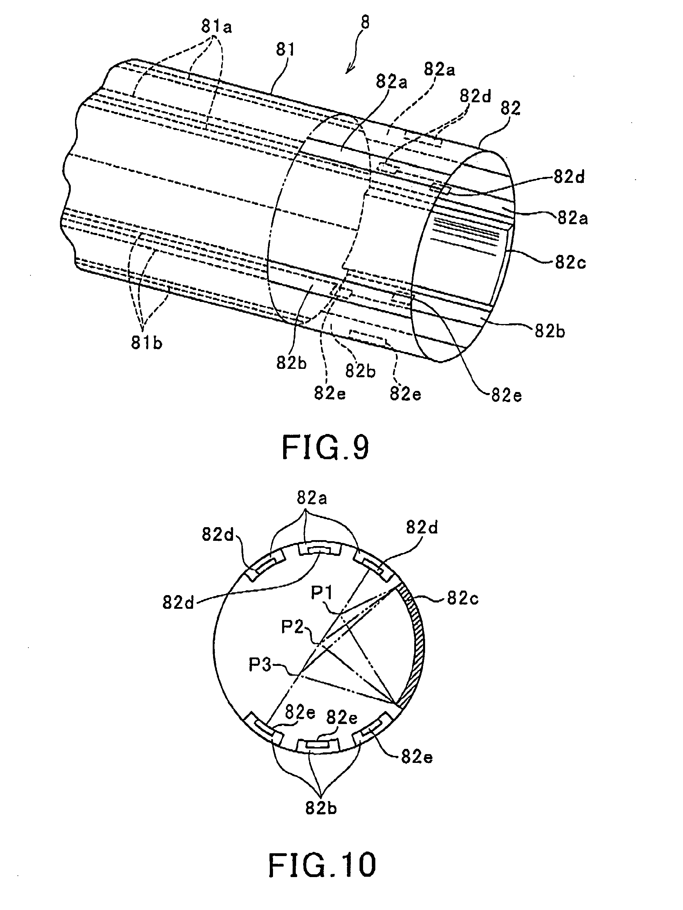

Referring to Figs. 9 and 10, a biological observation system according to a third embodiment of the present invention will now be described.

The biological observation system 1F according to the third embodiment concerns another configuration for radiating ultrasound waves toward a region being examined of an object, radiating light (a light beam) toward the region, and detecting modulated light from the region. The mechanism for the radiation and detection is arranged outside the endoscope, and is not limited to the configuration explained in the foregoing ones.

Practically, as shown in Fig. 9, there is provided a cylindrical sheath 8 which can be applied to the distal end of the elongated insertion tube of the endoscope so as to cover the distal end and part of the insertion tube. The mechanism for the radiation and detection is arranged within the sheath 8.

The sheath 8 comprises an approximately cylindrical sheath body 81 which is soft and flexible and adjusted in size to be applied to the distal end and a side part of the insertion tube and an approximate cylindrical cap which is rigid and detachably secured to the distal end.

Within a cylindrical bore of the sheath body 81, a plurality of light guide cables 81a and a plurality of light guide cables 81b are arranged. The light guide cables 81a are for transmitting, to the cap 82, the radiating light from the second light source unit 4 via the controller 5. In addition, the light guide cables 81b are for transmitting the light detected in the cap 82 to the controller 5.

Within the bore of the cap 82, there are arranged a plurality of light radiating members 82a located on the inner surface of the cap 82 at intervals in its circumferential direction, a plurality of light detecting members 82b located on the inner surface of the cap 82 at intervals in its circumferential direction, and an ultrasound emitting unit 82c. The light radiating members 82a are positionally opposed to the light detecting members 82b, respectively, via the center in a radial section of the bore. Each of the light radiating members 82a radiates the light transmitted through the assigned light guide cable 81a in a radial direction in the bore of the cap 82. Each light detecting member 82b detects (receives) the light transmitted radially from the opposing light radiating member 82a and outputs the detected light to the assigned light guide cable 81b. The ultrasound emitting unit 82c is placed to radially emit ultrasound waves in the bore of the cap 82 under the control of the ultrasound generator 7.

Each of the light radiating member 82a has a total reflection mirror 82d. This mirror 82d is arranged to reflect almost perpendicularly the light coming through each light guide cable 81a toward the opposing light detecting member 82b. At least, the front face of each light radiating member 82a is made of optically transparent material such as transparent plastic, so that the light radiated from the total reflection mirror 82d can be transmitted into the bore without optical interruption.

Each of the light detecting members 82b has a total reflection mirror 82e. This mirror is located to receive the light coming radially across the bore and reflect it almost perpendicularly toward the assigned light guide cable 81b. At least, the front face of each light detecting member 82b is made of optically transparent material such as transparent plastic, so that the light coming across the bore can be received without optical interruption.

In the example shown in Figs. 9 and 10, the light detecting members 82b and the light radiating members 82a are the same in number. However, this is not a definitive example. An alternative example is to use a single micromirror instead of the plural light radiating members 82a. The micromirror, which is driven by a not-shown driver placed in the controller 5, switches the direction of light reflected therefrom to each radial direction directed to each light detecting member 82b.

The ultrasound emitting unit 82c comprises layered components necessary for electrically scanning the ultrasound waved emitted therefrom. These components include, though not shown in Fig. 10, a backing material, an ultrasound transducer emitting ultrasound waves under the control of the ultrasound generator 7, matching layers, and an acoustic lens converging the ultrasound waves transmitted into the bore of the cap 82. Hence, responsively to delay time control from the ultrasound generator 7, the ultrasound emitting unit 82c is able to change the radiating direction (converged direction) and the converged position of the ultrasound waves emitted from the ultrasound transducer. For example, as shown in Fig. 10, the ultrasound emitting unit 82c is able to converge the ultrasound waves at each of plural points P1, P2, and P3 on each optical path connecting the mutually-opposed light radiating and detecting members 82a and 82b.

The operations of the present embodiment will now be described about a case where the foregoing sheath member 8 is applied to the endoscope described in the foregoing embodiments.

When being used, the sheath member 8 is applied to the insertion tube of the endoscope. The light guide cables 81a are electrically connected to the second light source units 4, the light guide cables 81b are electrically connected to the controller 5, signal lines from the ultrasound emitting unit 82c are electrically connected to the ultrasound generator 7. The sheath body 81 covers part of the insertion tube from the distal end thereof so that the cap 8 is located adjacently to the distal end of the insertion tube.

In the ultrasound modulated optical tomography mode, an operator manipulates the endoscope to insert, into a body cavity, the insertion tube equipped with the sheath member 8 so that the distal end of the insertion tube is located close to a region to be examined in the cavity. Then the operator pushes the insertion tube, that is, the sheath member 8, toward a body tissue including the region being examined. This makes it possible that the body tissue is forcibly taken into the cap 82, whereby the body tissue including the region being examined is fixedly located within the bore of the cap 82 for detection.

Under the control of the ultrasound generator 7, the ultrasound emitting unit 82c emits ultrasound waves toward the body tissue captured within the bore of the cap 82. The ultrasound waves produce an ultrasound converged region P1 (P2, P3) within the body tissue. Meanwhile, the light propagated through each the light guide cable 81a is radiated into the tissue body within the cap 82 via each light radiating member 82a. Within the body tissue, the radiated light passes the ultrasound converged region, resulting in that the radiated light is subjected to modulation on the ultrasound waves in the ultrasound converged region. This creates modulated light, which enters the light detecting member 82b spatially opposed to the member 82a radiating the radiating light.

The modulated light received by the light detecting member 82b is sent to the controller 5 via the light guide cable 81b, before being subjected to the processing in the controller 5 in a similar manner to the foregoing. Thus the modulated light is converted into video signals sent to the monitor 6, where tomographic images of the body tissue fixedly captured and supported within the cap 82 are displayed.

As the controller applicable to the present embodiment, the various controllers 5A - 5D already described with the modifications may be employed. Thus, the modulated light detected by the light detecting members 82b can be sent to any of those controllers 5A - 5D, with other types of modulation characteristic information can be acquired and displayed as tomographic images.

### (Fourth embodiment)

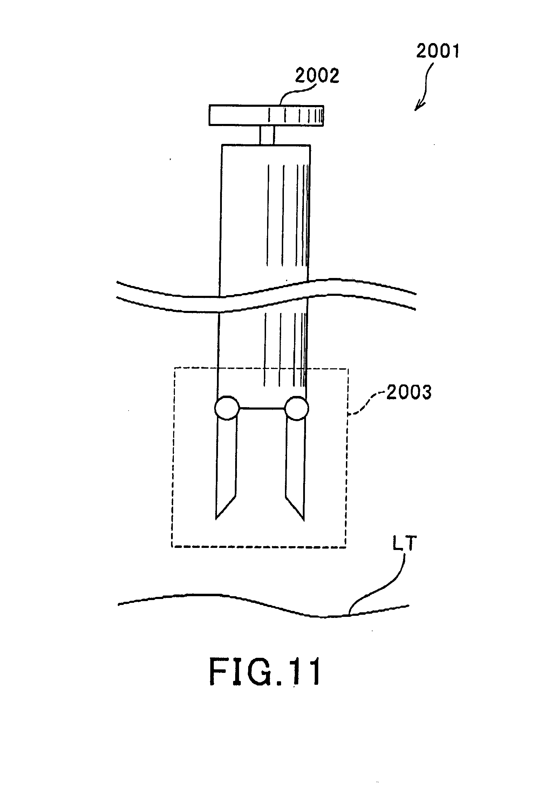

Referring to Figs. 11- 13, a biological observation system according to a fourth embodiment of the present invention will now be described.

In the present embodiment, a therapeutic instrument usable during observation on the endoscope has the configurations for emitting ultrasound waves toward a region to be examined, radiating light toward the region, and detecting modulated light from the region in the ultrasound modulated optical tomography mode.

By way of example, a therapeutic instrument 2001 shown in Fig. 11 is provided. This therapeutic instrument 2001 is provided with a lever 2002 to be manipulated and a therapeutic distal portion 2003. Manually manipulating the lever 2002 allows the therapeutic distal portion 2003 to grip a body tissue LT to be observed.

This therapeutic instrument 2001 is insertable into a therapeutic instrument channel of, for example, a conventional endoscope and shaped as a whole into an elongated form so that its therapeutic distal portion 2003 can be protruded from the tip of the insertion tube.

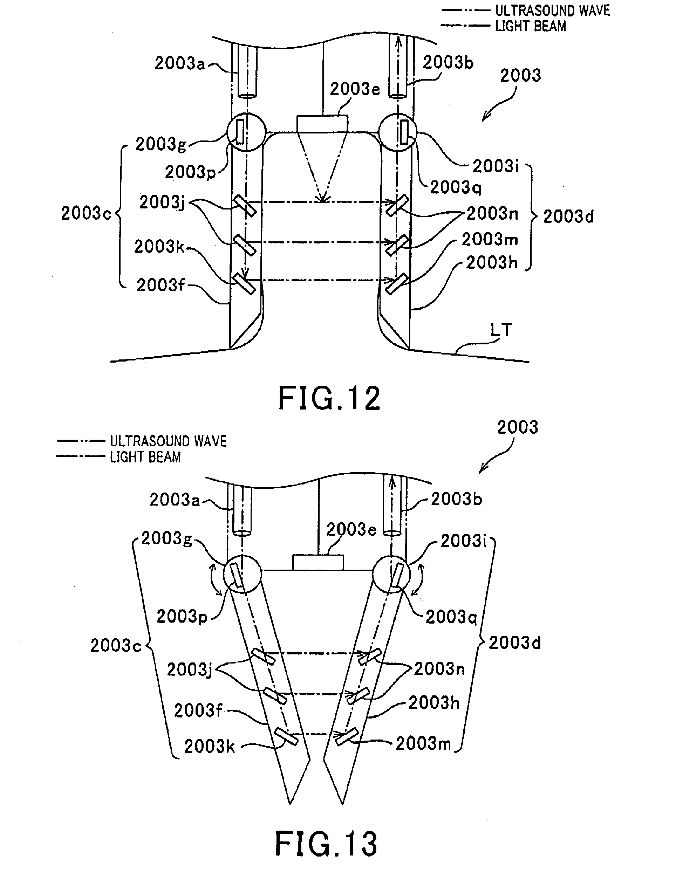

As shown in Figs. 12 and 13, at the therapeutic distal portion 2003, one end of each of light guide cables 2003a and 2003b is secured. Further, in this therapeutic distal portion 2003, an ultrasound emitting unit 2003e, a light radiating member 2003c, and a light detecting member 2003d. These light radiating and detecting members 2003c and 2003d are able to respond to operator's manual operations to allow its bar-shaped bodies to be tilted inward to come close to the longitudinal center of the therapeutic instrument 2001, as shown in Fig. 13.

The light guide cable 2003a is inserted within the therapeutic instrument 2001 and its end is located in front of the light radiating member 2003c in order to transmit the light coming from the second light source unit 4 to the light radiating member 2003c. Similarly, the other light guide cable 2003b is inserted within the therapeutic instrument 2001 and its end is located in front of the light detecting member 2003d in order to transmit the light received by the light detecting member 2003d to the controller 5.

The light radiating member 2003c comprises a bar-shaped mirror support 2003f and a joint 2003g coupled with the lever 2002 by a not-shown wire and movable to inwardly move the mirror support 2003f responsively to manual operations given to the lever 2002. The light radiating member 2003c composes part of the gripper of the therapeutic instrument 2001 and functions as a jaw for the therapeutic instrument 2001.

As shown in Fig. 12, the mirror support 2003f is arranged to radiate the radiating light propagated through the light guide cable 2003a toward the central axis of the therapeutic instrument 2001, which central axis is along the longitudinal direction thereof. The mirror support 2003f comprises therein a plurality of half mirror 2003j partly transmitting the radiating light and a total reflection mirror 2003k located at the last mirror position closest to the end of the therapeutic distal portion 2003. Thus the radiating light reflected by each of the mirrors 2003j and 2003k is transmitted toward the central axis. At least the wall portion of the mirror support 2003f, which faces the central axis, is made of an optically transparent material such as transparent plastic material, so that the radiating light beams from the mirrors 2003j and 2003k are not optically blocked.

The movable joint 2003g comprise therein a total reflection mirror 2003p, which is located to reflect the radiating light propagated through the light guide cable 2003a to the mirror support 2003f. Practically, as shown in Figs. 12 and 13, when the mirror support 2003f is in parallel with the central axis of the therapeutic instrument 2001 along the longitudinal direction thereof, the total reflection mirror 2003p does not reflect the radiating light from the light guide cable 2003a. In contrast, when the mirror support 2003f has a tilt to the central axis of the therapeutic instrument 2001, the total reflection mirror 2003p reflects the light depending on this tilt angle.

The light detecting member 2003d comprises a bar-shaped mirror support 2003h and a movable joint 2003i coupled with the lever 2002 by a not-shown wire and movable to inwardly move the mirror support 2003h responsively to manual operations given to the lever 2002. The light detecting member 2003d composes part of the gripper of the therapeutic instrument 2001 and functions as a jaw for the therapeutic instrument 2001.

As shown in Fig. 12, the mirror support 2003h comprises therein a total reflection mirror 2003m and a plurality of half mirrors 2003n. The total reflection mirrors 2003m reflects the incident light toward the end of the light guide cable 2003b. The half mirrors 2003n also reflect the respective incident light beams toward the light guide cable 2003b and allows the light from the total reflection mirror 2003m to pass therethrough toward the light guide cable 2003b. At least a wall portion of the mirror support 2003h, which faces the central axis, is made of an optically transparent material such as transparent plastic material, so that the incident light beams onto the mirrors 2003m and 2003n are not optically blocked.

The movable joint 2003i comprise therein a total reflection mirror 2003q, which is located to the detected light from the total reflection mirror 2003m and the respective half mirror 2003n to be propagated toward the light guide cable 2003b. Practically, as shown in Figs. 12 and 13, when the mirror support 2003h is in parallel with the central axis of the central axis of the therapeutic instrument 2001 along the longitudinal direction thereof, the total reflection mirror 2003q does not reflect the detected light from the mirrors. In contrast, when the mirror support 2003h has a tilt to the central axis of the therapeutic instrument 2001, the total reflection mirror 2003q reflects the light depending on this tilt angle.

The structure of the radiating-side mirror support 2003f will not be limited to that shown in Fig. 12, in which the number of mirrors of the mirror support 2003f is the same as those of the detecting-side mirror support 2003h and the positional correspondence between both mirror supports 2003f and 2003h is kept. For example, the mirror support 2003f may contain one micromirror driven by a driver (not shown) placed in the controller 5 such that the mircomirror reflects the radiating mirror toward each of the half mirrors and the total reflection mirror of the detecting-side mirror support 2003h.

The ultrasound emitting unit 2003e comprises a backing material, a ultrasound transducer, matching layers, and an acoustic lens, which are stacked on one another. The acoustic lens is able to converge the ultrasound waves in one direction in a region sectioned by the mirror supports 2003f and 2003h. Hence, under the delay time control of the ultrasound generator 7, the ultrasound emitting unit 2003e is able to electrically scanning the ultrasound waves (i.e., its emitting direction and converged position) in the region targeted.

The operations and advantages of the therapeutic instrument 2001 applied to the conventional endoscope will now be described.

The therapeutic instrument 2001 is used in the state where the light guide cables 2003a and 2003b are connected to the second light source unit 4 and the controller 5, respectively, and the signal line from the ultrasound emitting unit 2003e is electrically connected to the ultrasound generator 7.

When the ultrasound modulated optical tomography mode is designated, an operator inserts the insertion tube of the endoscope into a patient's body cavity and moves the distal end of the insertion tube to a position adjacent to a desired region to be examined. Then, the operator inserts the therapeutic instrument 2001 through the therapeutic instrument channel of the endoscope to make therapeutic distal portion 2003 protrude from the distal end of the endoscope. Then the operator manipulates the lever 2002 so that the light radiating and detecting members 2003c and 2003d cooperatively grip a body tissue LT of the region to be examined. Hence the light radiating and detecting members 2003c and 2003d and the ultrasound emitting unit 2003e are located to hold (support) the body tissue LT, as shown in Fig. 12, for example.

Under the delay time control of the ultrasound generator 7, the ultrasound emitting unit 2003e emits the ultrasound waves toward the body tissue LT along the central axis of the therapeutic instrument 2001. During this emission, the ultrasound emitting unit 2003e changes the direction and converted position of the emitted ultrasound waves so that each of the optical paths to be produced between the light radiating and detecting members 2003c and 2003d is scanned by the ultrasound waves at intervals.

The light propagated through the light guide cable 2003e is radiated from the light radiating member 2003c into the body tissue LT held (firmly supported) by both members 2003c and 2003d. The radiated light is then modulated by the ultrasound waves in the ultrasound converged region (position) from the ultrasound emitting unit 2003e. The modulated light is detected by the light detecting member 2003d.

The modulated light received by the light detecting member 2003d is transmitted to the controller 5 via the light guide cable 2003b. In the controller 5, the foregoing various processes are applied to the modulated light to be converted to video signals. The video signals are provided to the monitor 6 and displayed thereon as tomographic images of the body tissue LT, which is tightly held between the light radiating and detecting members 2003c and 2003d. The same operations are provided when the light radiating and detecting members 2003c and 2003d take the attitudes shown in Fig. 13.

Therefore, the present embodiment is still able to provide the identical or similar advantages to those gained in the foregoing embodiments and modifications. Moreover, the ultrasound modulated optical tomography can be conducted by effectively making use of the mobility of the therapeutic instrument itself.

## Claims

1. A medical apparatus for acquiring information indicative of an internal state of a region being examined of an object based on a modulated component of light caused by interaction with ultrasound waves in the region, the apparatus comprising:
light radiating means (4, 52f, 52a, 53a, 21b, 22e1) having a light radiating member (22g1) for radiating the light toward the region of the object;
light detecting means (22g2, 22e2, 21c, 53b) having a light detecting member (22g2) for detecting light that has been transmitted through the region of the object, the transmitted light being the light radiated from the light radiating member;
support means (22h, 59, 22f1, 22f2) for supporting both the light radiating member and the light detecting member so as to be opposed to each other with the region located between the light radiating and detecting members; and
ultrasound emitting means (22d, 7) having an ultrasound emitting member (22d) for emitting the ultrasound waves toward the region of object from a direction different from a radiating direction of the light radiated from the light radiating member, the emitted ultrasound waves modulating the radiated light.

2. The medical apparatus of claim 1, wherein the support means comprises
a first needle (22g1) equipped with the light radiating member and formed to be insertable into the region of the object; and
a second needle (22g2) equipped with the light detecting member and formed to be insertable into the region of the object.

3. The medical apparatus of claim 2, wherein the support member comprises adjusting means (22h, 59) for adjusting an amount of protrusion of the first and second needles from the medical apparatus in a state where the first and second needles are protruded in association with each other.

4. The medical apparatus of claim 2, wherein the first and second needles are arranged by a plurality of pairs each consisting of the first and second needles.

5. The medical apparatus of claim 1, wherein the support member comprises
a gripper that grips the region of the object;
a first jaw which is part of the gripper and which is provided with the light radiating member; and
a second jaw which is part of the gripper and which is provided with the light detecting member.

6. The medical apparatus of claim 1, comprising an endoscope having an insertion tube being insertable into the object,
wherein the light radiating member, the light detecting member, and the ultrasound emitting member are secured to the insertion tube.

7. The medical apparatus of claim 6, wherein
the ultrasound emitting means comprises means for converging the ultrasound waves emitted from the ultrasound emitting member in the region and spatially scanning a converged position of the ultrasound waves, and
the support means comprises position control means for positionally controls the light radiating and detecting members such that the light radiated from the light radiating member pass through the converged position of the ultrasound waves.

8. The medical apparatus of claim 7, wherein the support means are configured to be inserted within the region of the object to support the light radiating and detecting members.

9. The medical apparatus of claim 8, wherein the support means comprises
a first needle (22g1) equipped with the light radiating member and formed to be insertable into the region of the object; and
a second needle (22g2) equipped with the light detecting member and formed to be insertable into the region of the object.

10. The medical apparatus of claim 9, wherein
the first and second needles are arranged to be able to be protrude outward from the insertion tube in a direction crossing a longitudinal direction of the insertion tube, and insertable Into the region in a state where the light radiating and detecting members are opposed to each other.

11. The medical apparatus of claim 10, wherein the first and second needles are arranged by a plurality of pairs each consisting of the first and second needles.

12. The medical apparatus of claim 7, comprising means for acquiring images of sections of the region based on the modulated light detected by the detecting means.

13. The medical apparatus of claim 7, comprising
an endoscope having an insertion tube insertable into the object; and
a cap secured to a distal end of the insertion tube and formed to allow the region to be contained into the cap,
wherein the light radiating member, the light detecting member, and the ultrasound radiating member are secured to the cap.

14. The medical apparatus of claim 1, wherein the light radiating member, the light detecting member, and the ultrasound radiating member are secured to a therapeutic instrument being insertable into the object.

15. The medical apparatus of claim 14, wherein
the therapeutic instrument comprises the support member and
the support member comprises
a gripper that grips the region to be examined,
a first jaw composed of part of the gripper and provided with the light radiating member, and
a second jaw composed of part of the griper and provided with the light detecting member.
